(19) Europäisches Patentamt
European Patent Office
Office européen des brevets

(11) Publication number : **0 292 230 B1**

(12) **EUROPEAN PATENT SPECIFICATION**

(45) Date of publication of patent specification : **22.01.92 Bulletin 92/04**

(51) Int. Cl.$^5$ : **C07D 319/08,** C07D 417/08, C07D 501/04

(21) Application number : **88304442.2**

(22) Date of filing : **17.05.88**

(54) **Phenylacetic acid derivatives and their use in the preparation of cephalosporin antibiotics.**

(30) Priority : **18.05.87 JP 120947/87**
**18.05.87 JP 120948/87**

(43) Date of publication of application : **23.11.88 Bulletin 88/47**

(45) Publication of the grant of the patent : **22.01.92 Bulletin 92/04**

(84) Designated Contracting States : **DE FR GB IT**

(56) References cited :
**EP-A- 0 238 061**
**CHEMICAL ABSTRACTS, vol. 98, no. 23, June 6, 1983, Columbus, Ohio, USA, TAKEDA INDUSTRIES LTD,; "2-Iminothiazolidin-4-one derivatives" page 653, coumn 2, abstract-no. 198200e**
**CHEMICAL ABSTRACTS, vol. 92, no. 25, June 23, 1980, Columbus, Ohio, USA, MAGGIONI, P.; MINISCI, F.; "Aromatic hydroxy-and alkoxyaldehydes" page 588, column 2, abstract-no. 215 067n**

(73) Proprietor : **AJINOMOTO CO., INC.**
**5-8, Kyobashi 1-chome, Chuo-ku**
**Tokyo 104 (JP)**
Proprietor : **Mochida Pharmaceutical Co., Ltd.**
**1-7, Yotsuya Shinjuku-ku**
**Tokyo (JP)**

(72) Inventor : **Nakazawa, Masakazu**
**No. 1-1 Suzuki-cho Kawasaki-ku**
**Kawasaki-shi Kanagawa-ken (JP)**
Inventor : **Iwagami, Hisao**
**No. 1-1 Suzuki-cho Kawasaki-ku**
**Kawasaki-shi Kanagawa-ken (JP)**
Inventor : **Yatagai, Masanobu**
**No. 1-1 Suzuki-cho Kawasaki-ku**
**Kawasaki-shi Kanagawa-ken (JP)**
Inventor : **Hosoi, Akio**
**No. 1-1 Suzuki-cho Kawasaki-ku**
**Kawasaki-shi Kanagawa-ken (JP)**
Inventor : **Naora, Hirokazu**
**No. 1-1 Suzuki-cho Kawasaki-ku**
**Kawasaki-shi Kanagawa-ken (JP)**
Inventor : **Oonuki, Takashi**
**No. 1-1 Suzuki-cho Kawasaki-ku**
**Kawasaki-shi Kanagawa-ken (JP)**
Inventor : **Kato, Kazuo**
**No. 29-6, Fujimidai**
**Mishima-shi Shizuoka-ken (JP)**
Inventor : **Murakami, Kimihiro**
**No. 977-4, Kawashimata**
**Gotenba-shi Shizuoka-ken (JP)**

(74) Representative : **Armitage, Ian Michael et al**
**MEWBURN ELLIS & CO. 2/3 Cursitor Street**
**London EC4A 1BQ (GB)**

**EP 0 292 230 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel phenylacetic acid derivatives which are useful as intermediates for production of cephalosporin antibiotic substances.

M-14659, (see compound XIII infra) is under development for β-lactam antibiotics possessing good antibacterial activity (see "The 26th ICAAC Annual Meeting, September, 1986, New Orleans, U.S.A."). Such antibiotic possesses a complex structure, and thus is difficult to produce easily.

It is therefore desirable to have a method for producing this antibiotic industrially and advantageously and at low cost.

In one aspect the present invention provides a method for preparing phenylacetic acid derivatives of the general formula :

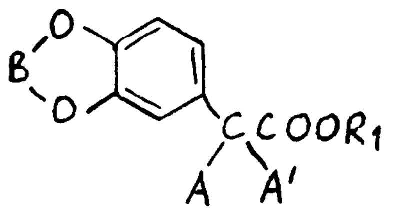

using catechol as the starting material. Such derivatives can be employed as intermediates for the production of antibiotic substances such as M-14659.

In the general formula mentioned above, A and A′ together may be oxygen, or A′ stands for hydrogen while A stands for hydroxy, halo such as chloro or bromo, phthalimidoxy, aminooxy, or a substituent of the formula:

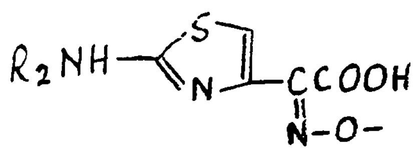

B stands for a phenolic hydroxy-protecting group, for example, ethylidene, propylidene such as iso-propylidene, butylidene such as 2-butylidene, pentylidene such as 3-pentylidene, cyclopentylidene and cyclohexylidene, two alkyl groups such as di-t-butyl, or two aralkyl groups such as di-benzyl ; $R_1$ stands for hydrogen, or a labile carboxyl-protecting group, for example, t-butyl, diphenylmethyl, p-methoxybenzyl or p-nitrobenzyl ; and $R_2$ stands for hydrogen or a labile amino-protecting group, for example, trityl or formyl.

Production of the derivatives of the present invention, and also production of antibiotic substances such as M-14659 using the derivatives as the starting material, can for example be carried out on the basis of the following steps.

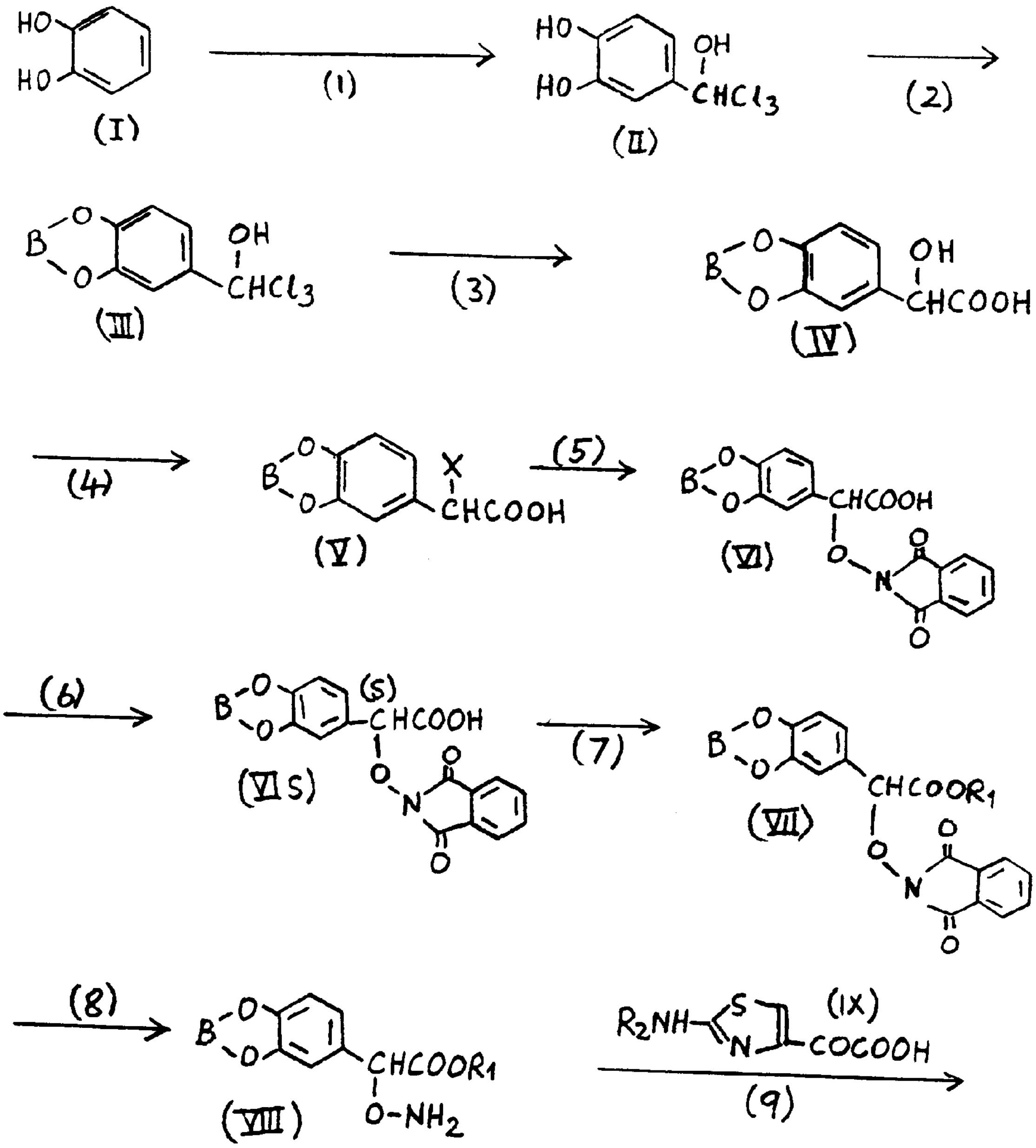


(I) (II) (III) (IV) (V) (VI) (VI S) (VII) (VIII) (IX)

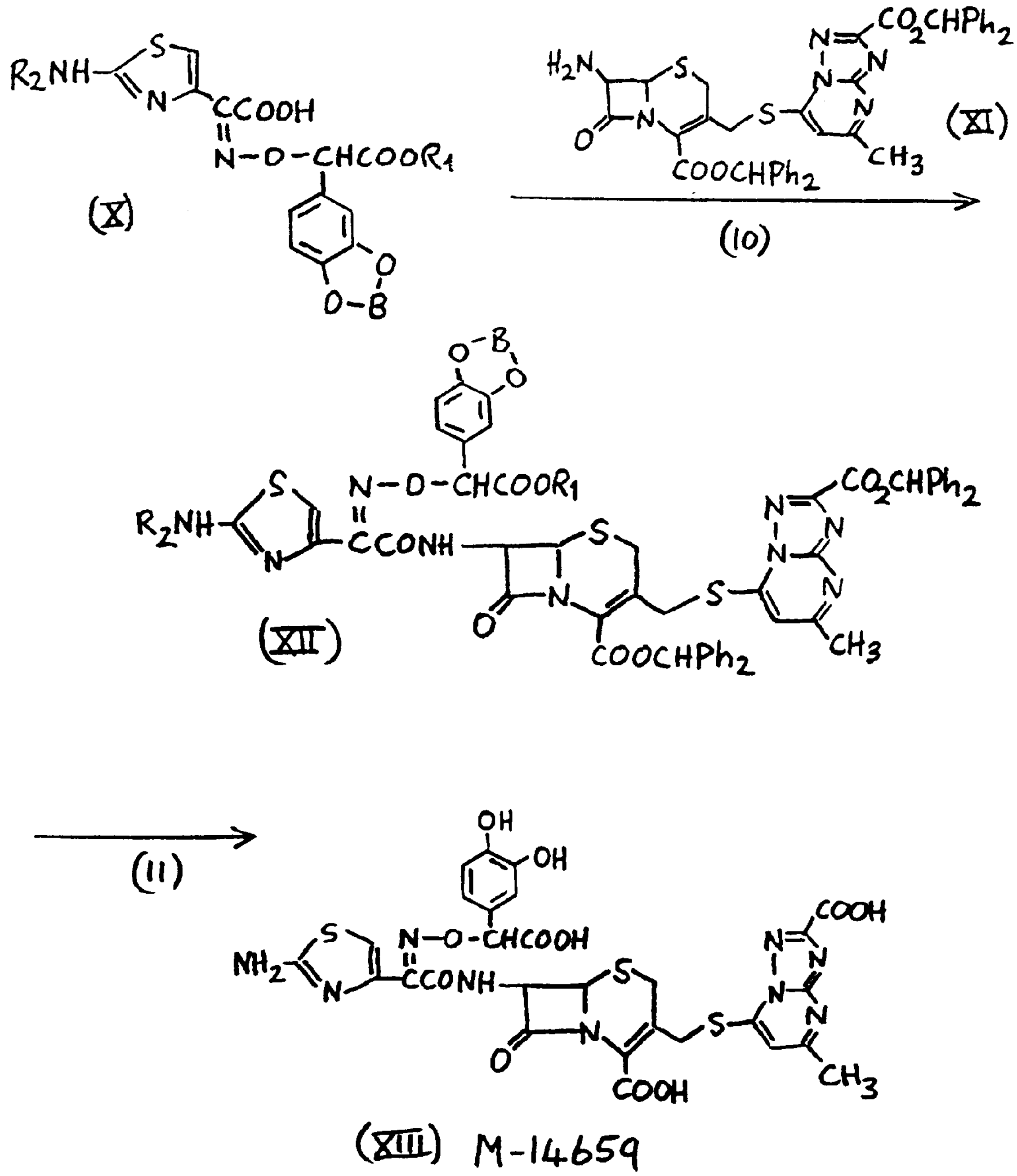

(X) (XI) (XII) (XIII) M-14659

In step (1), catechol and chloral are condensed together. For example, the reaction may be carried out in the presence of a base such as triethylamine. Bromal, in place of chloral, may be condensed to obtain 2-(3,4-dihydroxyphenyl)-2-hydroxy-1,1,1-tri-bromoethane.

In step (2), the phenolic hydroxyl groups are protected, suitably by a substance capable of being removed under acidic conditions. For example, a ketone corresponding to a desired protecting group, or a dialkyl acetal thereof, is reacted with compound II in the presence of an acid, to obtain compound III by dehydration or dealcoholation.

In step (3), hydrolysis is effected for example, using a base such as lithium hydroxide or sodium hydroxide in the presence of lithium chloride.

In step (4), the hydroxyl group is replaced by a halogen atom, using a halogenating reagent such as thionyl chloride, thionyl bromide, phosphorus pentachloride, phosphorus tribromide, triphenylphosphine-bromine complex, or other conventional halogenating reagents, to obtain the compound V (X = Cl, Br).

In step (5), the halogen atom is replaced by a phthalimidoxy group. N-hydroxyphtahlimide is reacted in the presence of a base such as sodium hydride and triethylamine to give the compound VI.

In step (6), optical resolution is effected, for example by forming a diastereisomer salt with an optically active amine, followed by fractional re-crystallization, and then reaction with a dilute acid to cleave the salt and obtain the (S) stereoisomer of compound VIS. The filtrate contains compound VI which is rich in the stereoisomer (R). This can be racemised by reaction with a Lewis acid such as aluminium trichloride and boron trifluoride etherate, and recovered.

In step (7), an ester is formed from the carboxyl group. This can be achieved by converting the carboxyl group to an acid halide, followed by alcoholysis with a desired alcohol. Alternatively the compound VI can be reacted with a desired diazo compound such as diphenyldiazomethane. The ester protective group is suitably capable of being removed in the presence of an acid catalyst.

Alternatively, esterification may be carried out earlier. For example, the compound IV can be reacted with a conventional hologenating reagent such as thionylchloride, thionyl bromide, phosphorous pentachloride, or phosphorous tribromide, and then treated with a desired alcohol to give an ester of the compound V ; and the compound VII can then be prepared in high yield by reacting the ester of compound V with N-hydroxyphthalimide in the presence of a base such as sodium hydride, and triethylamine.

In step (8), the phthalimidoxy group is converted into an aminooxy group by removing phthalic acid with hydrazine, amine, or the like.

In step (9), the compound VIII is reacted with 2-aminothiazol-4-yl glyoxylic acid derivative IX (see for example Japanese first patent publication No. 144786/1979 and No. 181270/1984) to obtain quantitatively 2-(2-aminothiazol-4-yl)-2-imino acetic acid derivative X.

The protective groups B, $R_1$ and $R_2$ are preferably capable of being removed under acidic conditions.

The compound VII can also be produced in the following manner.

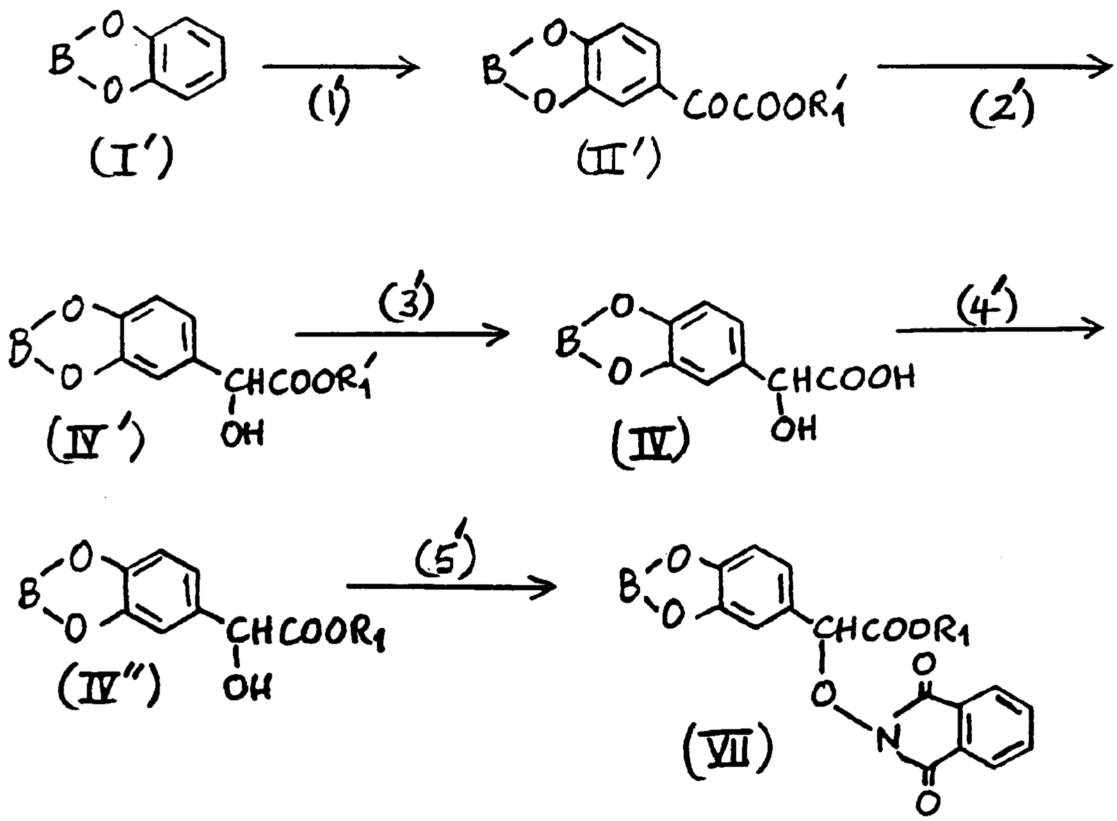


(I') (II') (IV') (IV) (IV'') (VII)

In the step (1'), catechol, (I') in which the phenolic hydroxyl groups have been protected, is reacted with alkyl chloroformyl formate such as ethyl chloroformyl formate in a Friedel-Crafts reaction to obtain the compound II'. In this case, $R'_1$ is a carboxyl protective group which can be hydrolyzed under basic conditions.

In the step (2'), the carbonyl group in the α-keto ester is reduced to the corresponding α-hydroxyester. If yeast is employed as reducing agent, the compound IV'R having the steroisomer (R) form at the chiral centre, is obtained. Likewise, if it is reduced with sodium borohydride in the presence of L-proline, compound IV' which

is rich in the (R)-stereoisomer is also obtained. However, if compound II′ is reduced merely with sodium borohydride, the racemate compound IV′ is obtained.

Step (3′) is a conventional hydrolysis of an ester. Step (4′) is esterification of the carboxylic group, and can be achieved in the same manner as in step (7) described above. Thus, in the ester IV″, it is desirable that $R_1$ is a protective group capable of being removed with an acidic catalyst.

In step (5′), the hydroxyl group is changed to a phthalimidoxy group. The compound VII can be obtained by a dehydration reaction of the compound IV″ and N-hydroxyphthalimide using azodicarboxylic acid dialkyl ester and triphenylphosphine. When the (R)-stereoisomer IV″ R of compound IV″ is employed, the configuration at the asymmetric carbon is inverted in the course of the dehydration reaction to obtain the compound VIIS of the (S)-configuration at the chiral centre.

Using the compound X as produced in the manner described above as the starting material, the antibiotic substance M-14659 can be produced by steps (10) and (11).

In step (10), the compound X and the compound XI are condensed by the action of dicyclohexylcarbodiimide, and the thus obtained condensation product may be purified by column chromatography.

The compound XI can be produced according to known methods (See "The 26th ICAAC Annual Meeting, September, 1986, New Orleans, U.S.A.". supra). For example, 7-aminocephalosporanic acid and 2-carboxy-7-mercapto-5-methyl-S-triazolo-[1,5-a] pyrimidine (see Japanese first patent publication No. 142987/1985) are reacted in the presence of a boron trifluoride ($BF_3$)-ether complex in acetonitrile, and the thus produced carboxylic acid derivative is reacted with diphenyldiazomethane to obtain the desired diphenylmethyl ester derivative in good yield.

In step (11), by treatment of compound XII with trifluoroacetic acid and anisole, the protective group B of the hydroxyl group in the catechol portion, the carboxy-protective group $R_1$, and the amino-protective group $R_2$, are all simultaneously removed to give the antibiotic substance M-14659.

Although the use of the novel intermediates hereof have been particularly exemplified in the production of antibiotic M-14659, it will be apparent that other related cephalosporin antibiotics will be preparable using these intermediates, for example by reaction with a variant of compound XI or by modification of the compound XIII.

EP-A-238061, a reference under Article 54(3) EPC, discloses the phenylacetic acid derivative 2-[α-benzhydryloxycarbonyl-3,4-di-(2-Methoxyethoxy Methoxy)-benzyloxyimino]-2-(tritylaminothiazol-4-yl) acetic acid.

## EXAMPLES

Hereinafter, the present invention will be further illustrated by the following examples and reference example.

### Example 1 2-(3,4-Dihydroxyphenyl)-2-hydroxy-1,1,1-trichloroethane (II)

Argon gas is bubbled through a mixture of catechol 110g (1 mol), chloral 160 g (1.1 mol) and 1,2-dichloroethane 200 ml. Triethylamine 20 g is added thereto under cooling with ice over about 20 minutes, the mixture is stirred for 5 minutes at room temperature, and then stirred for 3 hours at 50°C. After the completion of the reaction, 1,2-dichloroethane is distilled off using an evaporator. The residue is dissolved in ethyl acetate 1,3 l, and washed with 0.5 N HCl 400 ml. During this time the temperature rises, and therefore a small piece of ice is added. After such washing, it is washed with 200 ml water and then ethyl acetate is distilled off in the evaporator. To the residue 250 ml chloroform is added to crystallize the desired compound slowly. It is stood overnight at 5°C or below, and crystals are obtained on the filter paper and dried to give 130 g of the compound II in crystalline form. Melting point 123-125°C (dec.), yield about 50%.

Thin Layer Chromatography (TLC) Rf = 0.45 (Silica gel/Chloroform : methanol = 5 : 1)
$^1$H-NMR (90 MHz ; acetone-d6) δ ppm : 5.2 (1 H, d), 6.0 (1 H, d), 6.8 (1 H, d), 7.0 (1 H, d, d), 7.2 (1 H, d), 7.9 (1 H, S), 8.0 (1 H, S)

### Example 2 α-Trichloromethyl-3,4-0-isopropylidenedioxybenzyl alcohol (III : B = isopropylidene)

A mixture of a compound II 100 g (0.38 mol), 2,2-dimethoxy propane 58 ml (0.47 mool) and $P_20_5$ 0.6 g (3.8 mmol) in benzene (1 l) solution is heated under reflux in an oil bath. The reaction is carried out in a Soxhlet's extractor, and methanol by-product is removed with calcium chloride 150 g in an extraction thimble. After 2 hours 2,2-dimethoxypropane 15 ml (0.12 mol) is added. It is heated further under reflux for 3 hours. After completion of the reaction, the reaction solution is cooled to room temperature, and washed with 1 N sodium carbonate (400 ml × 2 times), saturated aqueous sodium chloride (200 ml × 2 times), and then dried over anhydrous magnesium sulfate. Next, silica gel 150 g is added and it is stirred for 10 minutes, and filtered. The

silica gel is washed with 1,2-dichloroethane (1 l), and the 1,2-dichloroethane solution is combined with the filtrate obtained previously, and the solvent is distilled off under reduced pressure to give the title compound III in an oily state yielding 80g (70%).
TLC, Rf = 0.72 (silica gel/ethyl acetate : n-hexane = 1 : 2)
$^1$H-NMR (90 MHz ; $CDCl_3$) δ ppm : 1.66 (6 H, S), 3.61 (1 H, d), 4.98 (1 H, d), 6.53-6.90 (3 H, m)
IR (Liquid film) ($cm^{-1}$) 3460, 2990, 2940, 1500, 1450, 1380, 1260, 820

Example 3 2-(3,4-0-Isopropylidenedioxyphenyl)-2-hydroxyacetic acid (IV : B = isopropylidene)

Lithium hydroxide 1 $H_2O$ (95%) 101 g (2.28 mol) is added to water 400 ml, and the mixture is cooled in an ice-water bath. The compound III 170 g (0.57 mol) as produced in Example 2 in dioxane (350 ml) solution is added while stirring and cooling. Thereafter, it is stirred for 3 days at room temperature.

After completion of the reaction, ice 200 g is added to the reaction solution. The mixture is made acidic with 6N hydrochloric acid aqueous solution 200 ml and ice-water 100 g is added. The mixture is stirred for 30 minutes and the precipitated crystals are separated by filtration. The crystals obtained on the filter paper are washed with water (1.51) and chloroform (0.71), and dried under reduced pressure to give the title compound IV as pale yellow crystals yielding 77 g (60%). M.P. 169-172°C (dec.)
TLC, Rf = 0.46 (Silica gel/methanol : chloroform = 1 : 3)
$^1$H-NMR (90 MHz ; DMSO-$d_6$) δ ppm : 1.61 (6 H, S), 4.85 (1 H, S), 6.60-6.83 (3 H, m) 8.2 (2 H, b)
IR (KBr, Tablet) ($cm^{-1}$) 3360, 2900, 2600, 1705, 1500, 1450, 1380, 1325, 1265

Example 4 2-(3,4-0-Isopropylidenedioxyphenyl)-2-bromoacetic acid (V : B = isopropylidene X = Br)

The compound IV 5 g (22.3 mmol) obtained in Example 3 is suspended in benzene 35 ml, and $PBr_3$ 5 ml (52.8 mmol) is added. It is refluxed under heating for 4 hours. After completion of the reaction, the reaction solution is cooled to room temperature, and added to a mixture of ice 100 g and ether 100 ml, and the mixture is stirred for 15 minutes. After stirring, the organic layer is separated and washed twice with water 100 ml, and with saturated aqueous sodium chloride 100 ml, and then dried over anhydrous magnesium sulfate. The solution is concentrated and the residue washed with ether-hexane to give the title compound V in the crystalline form, yielding 4.9 g. The compound consists of about 90% of the compound V and about 10% of the compound IV according to 'H-NMR analysis. Yield 69% (practical yield 4.4 g).
TLC, Rf = 0.61 (Silica gel/chloroform : Methanol = 3 : 1)
$^1$H-NMR (90 MHz ; DMSO-$d_6$) δ ppm : 1.63 (6 H, S), 5.43 (1 H, S), 6.5-7.0 (3 H, m)

Example 5 2-(3,4-0-Isopropylidenedioxyphenyl)-2-phthalimidoxy acetic acid (VI : B = isopropylidene)

N-Hydroxyphthalimide 2.3 g (14.1 mmol) is suspended in tetrahydrofuran (THF) 35 ml, and 60% oily suspended sodium hydride 1.25 g (31.2 mmol), which is employed after washing with hexane, added slowly while cooling in ice, and the mixture is stirred for 15 minutes.

The compound V 4.5 g (14.1 mmol, purity 90%) obtained in Example 4 is dissolved in THF 35 ml, and the solution is added dropwise and slowly to the above-described reaction solution under cooling with ice. After the dropwise addition, it is stirred for 3 hours under cooling in ice. After completion of the reaction, water 30 ml and ethyl acetate 50 ml are added, and then 2 N HCl is added to the solution under cooling in ice till the pH value of the solution is 1.5. The organic layer is separated, washed twice with water 50 ml and with saturated sodium chloride, and then dried over anhydrous magnesium sulfate and concentrated. It is not completely condensed. Ether 30 ml is added to the residue to crystallize it out slowly. The crystals are obtained on the filter paper to give the title compound VI yielding 3.9 g (73%).
M.P. 166-168°C
TLC, Rf = 0.52 (Silica gel/$CHCl_3$ : MeOH = 3 : 1)
$^1$H-NMR (90 MHz, DMSO-d6) δ ppm : 1.63 (6 H, S), 5.50 (1 H, s), 6.5-6.9 (3 H, m), 7.65 (4 H, S)
IR (KBr Tablet) $cm^{-1}$ : 3100, 1800, 1730, 1260

Example 6 (S)-2-(3,4-0-Isopropylidenedioxyphenyl)-2-phthalimidoxy acetic acid (VIS : B = isopropylidene)

Compound VI 1 g (2.7 mmol) obtained in Example 5 is mixed with quinine 880 mg (2.7 mmol) and ethanol-water (4 : 3) 7 ml. The solid matter is dissolved in the solvent at 70°C and the mixture is stood overnight. The precipitated crystals are obtained by filtration. To the crystals ethanol-water (5 : 1) 5 ml is added, and the solid matter is dissolved in the solvent at 70°C. The mixture is stood overnight. The precipitated crystals are

obtained by filtration. The crystals are dissolved in water, adjusted to pH 1.5, and the desired product is extracted with ethyl acetate washed with water and with saturated aqueous sodium chloride, and dried with anhydrous magnesium sulfate ($MgSO_4$). The solvent is distilled off, and to the thus obtained crystals, ether is added. The mixture is stirred and the crystals are obtained by filtration to give the title compound VIS yielding 0.15 g (15%).
Specific rotation $[\alpha]_D$ + 265.0° (c = 0.50, acetone)
M.P. 168-170°C
IR (KBr, Tablet) $cm^{-1}$ 3350, 1800, 1735, 1260

The compound VI, which is rich in (R)-form is recovered from the filtrate obtained from the optical resolution mentioned above. 3 gram of the compound VI is added to aluminium chloride 0.1 g in nitromethane (6 ml) solution while stirring and the mixture is stirred for 1.5 hours. Ethyl acetate 80 ml is added to the mixture, and the solution is washed with 1 N HCl, and with saturated NaCl solution, and then dried. The solvent is distilled off. To the residue ethyl acetate 6 ml is added, and the mixture is stirred for 30 minutes at room temperature. The mixture is filtered. The crystals are washed with 1,2-dichloroethane to obtain the compound VI as the racemate, yielding 1.8 g.

Example 7 (S)-2-(3,4-0-Isopropylidenedioxyphenyl)-2-phthalimidoxy acetic acid tert-butyl ester (VII : B = isopropylidene, $R_1$ = tert-butyl)

The compound VIS 1.0 g (2.71 mmol) obtained in Example 6, and pyridine 0.44 ml (5.44 mmol) in toluene (15 ml) solution is cooled in an ice-water bath, and thionyl chloride 0.40 ml (5.50 mmol) in toluene (5 ml) solution is added dropwise over a period of about 5 minutes to the mixture while cooling and stirring. At the end of the dropwise addition, it is stirred for 5 minutes, and then for 45 minutes at room temperature. After completion of the reaction, the solvent and unreacted thionyl chloride are distilled off under reduced pressure, and then toluene (5 ml) is added. The solvent is also distilled off under reduced pressure.

To the residue, toluene 10 ml and pyridine 0.87 ml (0.01 mol) are added, and the mixture is cooled in an ice-water bath. Toluene (5 ml) solution of tert-butyl alcohol 1.1 ml (0.01 mol) is added dropwise over a period of 5 minutes to the solution while cooling and stirring in the ice-water bath. It is stirred for 5 minutes in the ice-water bath, and then further stirred for 5 minutes at room temperature. After completion of the reaction, the reaction solution is poured into ice-water 30 ml, and the desired product is extracted with ethyl acetate 30 ml. The organic layer is washed with 1 N HCl (10 ml × twice), $NaHCO_3$-saturated solution (20 ml × twice) and NaCl-saturated solution in the order as described, and dried with anhydrous magnesium sulfate. The solvent is distilled off under reduced pressure to obtain the title compound VII as pale yellow solid, yielding 0.81 g (70%).
Specific rotation $[\alpha]_D$ + 154.0° (C = 0.20, $CHCl_3$)
M.P. 100-104.5°C
TLC, Rf = 0.50 (Silica gel/ethyl acetate : n-hexane = 1 : 2)
$^1$H-NMR (90 MHz, $CDCl_3$) $\delta$ ppm = 1.46 (9 H, S), 1.66 (6 H. S), 5.60 (1 $H_1$S), 6.58-7.00 (3 H, m), 7.60-7.83 (4 H. m)
IR (KBr) ($cm^{-1}$) 2990, 2930, 1795, 1740, 1500, 1450, 1375, 1260, 1150, 700

Example 8 2-(3,4-0-Isopropylidenedioxyphenyl)-2-phthalimidoxyacetic acid tert-butyl ester (VII : B = Isopropylidene, $R_1$ = tert-butyl)

2-(3,4-0-Isopropylidene dioxyphenyl)-2-hydroxy acetic acid (IV) 30 g (0.13 mol), and toluene (200 ml) solution in DMF 31 ml (0.4 mol) are cooled in an ice-water bath, and toluene (100 ml) solution of thionyl chloride 29 ml (0.4 mol) is added dropwise over a period of 30 minutes to the solution while stirring. Thereafter, it is stirred for 3 hours at room temperature. After completion of the reaction, benzene 300 ml is added. The mixture is washed speedily with ice-water (300 ml × twice), and with saturated NaCl (300g × twice) and then dried with anhydrous magnesium sulfate. The solvent is distilled off under reduced pressure, and thereby the solution is concentrated to about 100 ml or so. The thus concentrated solution is added dropwise over a period of 10 minutes to a mixture of tert-butanol 50 ml (0.52 mol), benzene (100 ml) and pyridine 43 ml (0.53 mol) while cooling and stirring in the ice-water bath. The mixture is further stirred for 1 hour at room temperature, and ethyl acetate 200 ml is added thereto. It is washed with ice-water (400 ml), 0.5 N HCl (400 ml × 3 times), dilute $NaHCO_3$ aqueous solution (400 ml), and NaCl-saturated aqueous solution (400 ml), and then dried with anhydrous magnesium sulfate. The solvent is distilled off under reduced pressure. From the residue the desired product is extracted with n-hexane 500 ml. The solvent is distilled off under reduced pressure to obtain 2-(3,4-0-isopropylidenedioxyphenyl)-2-chloroacetic acid tert-butyl ester in crystalline form, yielding 30 g (76%).
M.P. 60-62°C,

TLC, Rf = 0.65 (Ethyl acetate : n-hexane = 1 : 5)
$^{1}$H-NMR (90 MHz, $CDCl_3$) δ 1.43 (9 H, S), 1.65 (6 H, S), 5.03 (1 H, S), 6.48-6.78 (3 H, m)
IR (KBr) ($cm^{-1}$) 2990, 2930, 1740, 1505, 1450, 1380, 1370, 1265, 1145

2-(3,4-0-isopropylidenedioxyphenyl)-2-chloroacetic acid tert-butyl ester 70 g (0.23 mol) obtained as above, is dissolved in acetonitrile 200 ml, and the mixture is cooled in an ice-water bath. N-hydroxyphthalimide 38 g (0.23 mol) and triethylamine 33 ml (0.23 mol) in acetonitrile (200 ml) are added dropwise over a period of about 30 minutes to the above mixture under cooling. After the dropwise addition, the mixture is stirred for 12 hours at 40°C in an oil bath. After completion of the reaction, ethyl acetate 500 ml is added, and the mixture is washed with ice-water (500 ml × twice), saturated $NaHCO_3$ (200 ml × twice), and saturated NaCl (300 ml) in that order, and then dried over anhydrous magnesium sulfate. The solvent is distilled off under reduced pressure. The residue is dissolved in a mixture of ether 300 ml and n-hexane 1 l to precipitate crystals. The crystals are separated by filtration, and the filtrate is concentrated to obtain further crystals. Both crystals are mixed together to give the title compound VII as crystals, yielding 82 g (81%). M.P. 121.0-121.5°C.
TLC, Rf = 0.50 (Silica gel/ethyl acetate : n-hexane = 1 : 2)
$^{1}$H-NMR (90 MHz, $CDCl_3$) δ ppm : 1.46 (9 h, S), 1.65 (6 H, S), 5.60 (1 H, S), 6.58-7.00 (3 H, m), 7.58-7.81 (4 H, m)
IR (KBr Tablet) ($cm^{-1}$) 2980, 2940, 1795, 1740, 1495, 1450, 1370, 1255, 1155, 710

Example 9 2-(3,4-0-isopropylidenedioxyphenyl)-2-phthalimidoxy acetic acid diphenylmethyl ester (VII : B = isopropylidene, $R_1$ = diphenylmethyl)

The compound VI 1.0 g (2.71 mmol) obtained in Example 5 in ethyl acetate (20 ml) solution is prepared, and to the solution while stirring at room temperature, ethyl acetate (20 ml) solution in diphenyl diazomethane 0.53 g (2.73 mmol) is slowly added dropwise. After the dropwise addition, the mixture is stirred for 1 hour at room temperature. After completion of the reaction the solvent is distilled off under reduced pressure. The residue is washed with n-hexane (20 ml), and dried under reduced pressure to obtain the title compound VII as a white solid, yielding 1 g (69%).
TLC, Rf = 0.53 (Silica gel/ethyl acetate : n-hexane = 1 : 2)
$^{1}$H-NMR (90 MHz, $CDCl_3$) δ ppm 1.61 (6 H, S), 5.80 (1 H, S), 6.46-7.13 (14 H, m), 7.56 (4 H, m)
IR (KBr, tablet) ($cm^{-1}$) 3170, 3130, 2990, 2940, 1795, 1735, 1495, 1450, 1375, 1255, 1185, 700

Example 10 (S)-2-(3,4-0-isopropylidenedioxyphenyl)-2-aminooxy acetic acid tert-butyl ester (VIII : B = Isopropylidene, $R_1$ = tert-butyl)

(S)-2-(3,4-0-isopropylidenedioxyphenyl)-2-phthalimidoxy acetic acid tert-butyl ester (VII) 0.91 g (2.14 mmol) is dissolved in methylene chloride 15 ml, and the mixture is cooled in an ice-water bath.

Hydrazine hydrate 0.21 ml (4.33 mmol) is added to the solution while cooling and stirring, and the mixture is stirred for 1 hour. After completion of the reaction, the precipitated matter is separated by filtration. The filtrate is dried over anhydrous magnesium sulfate. The solvent is distilled off under reduced pressure to give the title compound VIII in the optically active form as pale yellow solid, yielding 0.44 g (70%).
Specific rotation [α] D + 35.0 (c = 0.20, $CHCl_3$)
M.P. 48.5-52.5°C
TLC, Rf = 0.50 (Silica gel/ethyl acetate : n-hexane = 1 : 2)
$^{1}$H-NMR (90 MHz, $CDCl_3$) δ ppm 1.46 (9 H, S), 1.65 (6 H, S), 4.16 (1 H, S), 5.66 (2 H, b), 6.53-6.83 (3 H, m)
IR (KBr, tablet) ($cm^{-1}$) 3320, 3250, 2990, 2970, 2930, 1735, 1500, 1450, 1385, 1380, 1370, 1260, 1245, 1220, 1160

Example 11 2-(3,4-0-isopropylidenedioxyphenyl)-2-aminooxy acetic acid tert-butyl ester (VIII : B = isopropylidene, $R_1$ = tert-butyl)

Compound VIII is produced (yield : 75%) in the same manner as in Example 10 using 2-(3,4-0-isopropylidenedioxyphenyl)-2-phthalimidoxy acetic acid tert-butyl ester (VIII) as starting material.
M.P. 56.0-57.5°C
TLC, Rf = 0.50 (Silica gel/ethyl acetate : n-hexane = 1 : 2)
$^{1}$H-NMR (90 MHz, $CDCl_3$) δ ppm 1.46 (9 H, S), 1.65 (6 H, S), 4.16 (1 H, S), 5.66 (2 H, b), 6.53-6.83 (3 H, m)
IR (KBr, Tablet) ($cm^{-1}$) 3320, 3260, 2990, 2980, 2940, 1735, 1500, 1450, 1390, 1380, 1370, 1255, 1210, 1155

Example 12 α-trichloromethyl-3,4-0-cyclohexylidenedioxybenzyl alcohol (III : B = cyclohexylidene)

A mixture of the compound II 5 g (19.4 mmol) and 1,2-dichloroethane 40 ml is heated at 70°C in an oil bath. To the solution while heating and stirring, 1,2-dichloroethane (10 ml) solution of cyclohexanone 2.4 ml (23.1 mmol) and $P_2O_5$ 1.4 g (9.8 mmol) are each divided into 5 portions and added alternately over a period of about 1 hour.

It is stirred further under heating for 4 hours, and then the reaction mixture is cooled to room temperature, and the thus obtained precipitate is obtained by decantation. The precipitate is washed with chloroform 30 ml. The chloroform solution and the above reaction solution are mixed together, and washed with 1 N $Na_2CO_3$ (50 ml × 3 times) and NaCl-saturated aqueous solution (50 ml), and dried with anhydrous magnesium sulfate. Next, the solvent is distilled off under reduced pressure, and the mixture is concentrated to about 3 ml. Silica gel 10g is added, and the mixture is stirred for 10 minutes, and filtrated. The silica gel is washed with chloroform 30ml. The previously obtained filtrate and thus washed solution are combined together. The solvent is distilled off under reduced pressure. To the residue n-hexane 20 ml is added to crystallize the desired product. The crystals are obtained by filtration, and washed with n-hexane 20 ml, and dried under reduced pressure to obtain the title compound III as white crystals, yielding 2.4 g (32%).

M.P. 105-108°C

TLC, Rf = 0.60 (Silica gel/ethyl acetate : n-hexane = 1 : 2)

$^1$H-NMR (90 MHz, $CDCl_3$) δ ppm : 1.3-2.0 (10 H, m), 3.20 (1 H, d), 5.03 (1 H, d), 6.43-6.96 (3 H, m)

IR (KBr Tablet) ($cm^{-1}$) 3520, 2940, 2870, 1500, 1450, 1265, 1060, 825

Example 13 2-(3,4-0-cyclohexylidenedioxyphenyl)-2-hydroxyacetic acid (IV : B= cyclohexylidene)

To LiOH 1 $H_2O$ (95%) 5.2 g (0.117 mol) water 20 ml is added, and to the mixture while cooling and stirring in an ice-water bath, the compound III 11.2 g (0.029 mol) prepared in Example 12 in dioxane (20 ml) solution is added. Thereafter, the mixture is stirred for four days at room temperature.

After completion of the reaction, ice 15 g is added to the reaction solution. The solution is made acidic by the addition of 6 N HCl 11 ml, and stirred for 30 minutes. The thus precipitated crystals are separated by filtration. The crystals obtained on the filter paper, are washed with water 200 ml and chloroform 100 ml, and then dried under reduced pressure to obtain the title compound IV as pale yellow crystals, yielding 5.3 g (yield = 59%).

M.P. 164-166°C

TLC, Rf = 0.49 (Silica gel/methanol : chloroform = 1 : 3)

$^1$H-NMR (90 MHz, DMSO-$d_6$) δ ppm = 1.3-2.0 (10 H, m) 4.71 (1 H, S), 6.57-6.77 (3 H, m), 8.2 (2 H, b)

IR spectrum (KBr, tablet) ($cm^{-1}$) 3430, 2940, 2870, 1710, 1500, 1450, 1285, 1255

Example 14 2-(3,4-0-cyclohexylidenedioxyphenyl)-2-bromoacetic acid (V : B = cyclohexylidene, X = bromine)

The compound IV 1.5 g (4.8 mmol) obtained in Example 13 is suspended in benzene 10 ml, and $PBr_3$ 1.1ml (11.5 mmol) is added. The mixture is refluxed under heating for 4 hours. After completion of the reaction, the reaction solution is cooled to room temperature, and added to a mixture of ice 20 g and diethyl ether 20ml. The mixture is stirred for 15 minutes. The organic layer is separated, washed with water (20 ml × twice) and saturated NaCl (20 ml) and then dried over anhydrous magnesium sulfate.

The solvent is distilled off under reduced pressure. The residue is washed with diethyl ether/n-hexane, and dried under reduce dpressure to obtain the title compound V as pale yellow crystals yielding 1.2 g. It gives about 90% of the compound V, and about 10% of the compound IV from $^1$H-NMR analysis, yielding 63% (practical yield : 1.1 g).

TLC, Rf = 0.76 (Silica gel/methanol : chloroform = 1 : 3)

$^1$H-NMR (90 MHz, DMSO-d6) δ ppm = 1.3-2.0 (10 H, m), 5.56 (1 H, S), 6.66-7.00 (3 H, m)

IR spectrum (KBr, tablet) ($cm^{-1}$) : 3430, 2950, 2860, 1725, 1500, 1450, 1285, 1255

Example 15 2-(3,4-0-cyclohexylidene dioxyphenyl)-2-phthalimidoxy acetic acid (VI : B = cyclohexylidene)

N-hydroxyphthalimide 0.42 g (2.57 mmol) is suspended in tetrahydrofuran (THF) 7 ml, and 60% oily suspended sodium hydride 0.23 g (5.75 mmol) is added slowly thereto. The mixture is stirred for 15 minutes.

The compound V 1.0 g (2.56 mmol ; purity : 90%) obtained in Example 14 is dissolved in THF 7 ml, and the thus obtained THF solution is slowly added dropwise while cooling with ice. After the dropwise addition, it

is stirred for 3 hours under cooling the ice, and then for 17 hours at room temperature. After completion of the reaction, water (10 ml), and ethyl acetate (20 ml) are added to the mixture. 1 N hydrochloric acid aqueous solution is added to the mixture under cooling with ice until the pH of the solution is 1.5. The organic layer is separated, and washed with water (20 ml × twice) and saturated NaCl (20 ml), and dried with anhydrous magnesium sulfate. The solution is concentrated and ether (10 ml) is added to crystallize the desired material. The crystals are obtained by filtration and dried in air to obtain the title compound VI as pale yellow crystals, yielding 0.45g (41%).
M.P. 160-161°C
TLC, Rf = 0.58 (silica gel/methanol : chloroform = 1 : 3)
$^1$H-NMR (90 MHz, DMSO-$d_6$) δ ppm : 1.3-2.0 (10 H, m), 5.58 (1 H, S) 6.68-6.95 (3 H, m), 7.75 (4 H, S)
IR spectrum (KBr, tablet) ($cm^{-1}$) 3440, 2950, 2860, 1790, 1740, 1500, 1450, 1260, 705.

Example 16 2-(3,4-0-cyclohexylidenedioxyphenyl)-2-phthalimidoxy acetic acid tert-butyl ester (VII : B = cyclohexylidene, $R_1$ = tert-butyl)

The compound VI 0.35 g (0.81 mmol) obtaind in Example 15 and pyridine 0.13 ml (1.61 mmol) in toluene (5 ml) solution are cooled in an ice-water bath. Thionyl chloride 0.12 ml (1.65 mmol) is added to the mixture while cooling and stirring in the ice-water bath. The mixture is stirred for 2 hours at room temperature, and the solvent and unreacted thionyl chloride are distilled off under reduced pressure. Further, toluene 2 ml is added thereto, and the solvent is also distilled off under reduced pressure. To the residue toluene 5 ml and pyridine 0.26 ml (3.21 mmol) are added. To the thus obtained mixture while cooling and stirring in the ice-water bath, tert-butyl alcohol 0.3 ml (3.17 mmol) in toluene (2 ml) solution is slowly added dropwise. The mixture is stirred for 10 minutes at room temperature, and the thus reacted mixture is poured into water 20 ml. The desired product is extracted with ethyl acetate 30 ml. The organic layer is washed with 1 N HCl (10 ml × twice) satured $NaHCO_3$ (20 ml) and satured NaCl (20 ml × twice) in that order, and dried over anhydrous magnesium sulfate. The solvent is distilled off under reduced pressure. From the residue the desired product is extracted with ether-hexane (1 : 3) 6 ml. The solvent is distilled off under reduced pressure to give the title compound VII as pale yellow solid, yielding 0.22 g (55%).
TLC, Rf = 0.60 (silica gel/ethyl acetate : n-hexane = 1 : 2)
$^1$H-NMR (90 MHz, $CDCl_3$) δ ppm : 1.45 (9 H, S), 1.3-2.0 (10 H, m), 5.56 (1 H, S), 6.58-6.96 (3 H, m), 7.71 (4 H, m)
IR spectrum (KBr, tablet) ($cm^{-1}$) : 2940, 2870, 1740, 1500, 1450, 1370, 1255, 1155, 710

Example 17 2-(3,4-0-cyclohexylidenedioxyphenyl)-2-aminooxy acetic acid tert-butyl ester (VIII : B = cyclohexylidene, $R_1$ = tert-butyl)

The compound VII 0.19 (0.38 mmol) obtained in Example 16 is dissolved in methylene chloride 5 ml. To the mixture while cooling and stirring in an ice-water bath hydrazine hydrate 0.038 ml (0.78 mmol) is added. The mixture is stirred for 30 minutes. After a completion of the reaction the thus precipitated material is separated by filtration. The thus obtained filtrate is dried over anhydrous magensium sulfate and the solvent is distilled off under reduced pressure. From the residue the desired material is extracted with ether-hexane (1 : 3) 5 ml, and the solvent is distilled off under reduced pressure to give the title compound VIII as a pale yellow oily substance, yielding 72 mg (49%).
TLC, Rf = 0.53 (silica gel/ethyl acetate : n-hexane = 1 : 2)
$^1$H-NMR (90 MHz, $CDCl_3$) δ ppm : 1.5 (19 H, m) 4.83 (1 H, S), 5.6 (2 H, b), 6.55-6.80 (3 H, m)
IR spectrum (liquid film) ($cm^{-1}$) 3330, 3260, 2940, 2870, 1740, 1500, 1445, 1370, 1340, 1280, 1250, 1155, 1060

Example 18 3,4-0-isopropylidenedioxyphenylglyoxylic acid ethyl ester (II′ = B = isopropylidene, $R_1'$ = ethyl)

Aluminium chloride ($AlCl_3$) 37.5 g (282 mmol) is suspended in dichloromethane 300 ml, and the mixture is cooled to 0°C. Ethyl chloroformyl formate 25.0 g (183 mmol) is added thereto, and the mixture is stirred for a while. 1,2-0-isopropylidenedioxybenzene 27.0 g (180 mmol) in dichloromethane (80 ml) is added dropwise over a period of 1 hour to the above mixture, and the mixture is stirred for 1 hour at room temperature. The reaction mixture is poured into broken ice 200 g, and then the organic layer is separated. From the aqueous layer, the desired material is extracted twice with dichloromethane 100 ml. The combined organic layers are washed with water, and then dried over magnesium sulfate. The solvent is distilled off under reduced pressure to give a red-coloured liquor of the title compound II′ (B = isopropylidene, R′ = ethyl), yielding 41.2 g.
$^1$H-NMR (90 MHz ; chloroform-$d_1$) δ ppm : 1.4 (3 H, t), 1.7 (6 H, S), 4.3 (4 H, quar), 6.7 (1 H, d), 7.2-7.5 (3 H,

m)

Example 19 (R)-2-(3,4-0-isopropylidenedioxyphenyl)-2-hydroxyacetic acid ethyl ester (IV′R : B = isopropylidene, $R_1'$ = ethyl)

Sucrose 15 g, distilled water 100 ml, and living yeast 14 g are respectively put into thirteen 500 ml Sakaguchi flasks. Each mixture is stirred for 1 hour at 30°C. The compound II′ (0.60 g) as obtained in Example 18 is added. The mixture is further stirred for 22 hours at 30°C, and filtrated through cellite. The cellite is washed with water and ethyl acetate. The filtrate of the ethyl acetate layer is separated. The desired product is extracted with ethyl acetate from the aqueous layer. The thus obtained ethyl acetate layers are combined together, and washed with saturated NaCl and dried over magnesium sulfate. The solvent is distilled off under reduced pressure. The thus obtained product is purified by silica gel column chromatography (hexane : ethyl acetate = 6 : 1) to give the title compound IV′R as pale yellow liquor, yielding 5.14 g.
Specific rotation $[\alpha]_D$ 17.0-82.50 (C = 1.03, $CHCl_3$)
$^1$H-NMR (90 MHz, chloroform-$d_1$) δ ppm = 1.2 (3 H, t), 1.65 (6 H, S), 3.3 (1 H, broad d), 4.0-4.3 (2 H, m), 4.9 (1 H, broad d), 6.5-6.8 (3 H, m)

Sodium borohydride 38 mg (1.0 mmol) and L-proline 115 mg (1.0 mmol) are suspended in THF 2.0 ml and the mixture is stirred for 3.5 hours at room temperature. In this time, the liquor becomes very transparent. After the flask is cooled to 0°C, THF (0.2 ml) solution in which the compound II′ 250 mg (1.0 mmol) from Example 18 has been dissolved, is put into the cooled flask. The mixture is stirred for 17.5 hours at 0°C. Water 5 ml is added thereto, and the desired product is extracted three times with ethyl acetate (5 ml). The organic layer is washed with 1 M HCl, saturated $NaHCO_3$ and saturated NaCl in that order, and dried with anhydrous magnesium sulfate. The solvent is distilled off under reduced pressure to obtain the title compound IV′R as pale yellow liquor, yielding 184 mg.
Specific rotation $[\alpha]_D$ 21.5 = – 44.6° (C = 0.965, $CHCl_3$)

Example 20 (R)-2-(3,4-0-isopropylidenedioxyphenyl)-2-hydroxyacetic acid (IVR : B = isopropylidene)

The compound IV′R 0.68 g (2.69 mmol) obtained by reduction with yeast as described in Example 19 is dissolved in ethanol 7 ml. To the mixture while cooling and stirring in an ice-water bath, 1 N KOH 3.2 ml is added. The mixture is stirred for 30 minutes, and then adjusted to pH 2.5 by the addition of hydrochloric acid solution. Water 20 ml is added and the desired product is extracted with ethyl acetate 100 ml. The organic layer is washed with saturated NaCl solution, and dried over anhydrous magnesium sulfate. The solvent is distilled off under reduced pressure to obtain the title product, yielding 0.55 g (91%).
TLC Rf = 0.46 (silica gel/methanol : chloroform = 1 : 3)
$^1$H-NMR (90 MHz ; DMSO-$d_6$) δ ppm : 1.61 (6 H, S), 4.85 (1 H, S), 6.60-6.83 (3 H, m), 8.2 (2 H, b)

Example 21 (R)-2-(3,4-0-isopropylidenedioxyphenyl)-2-hydroxyacetic acid diphenylmethyl ester (IV″R : B = Isopropylidene, $R_1$ = diphenylmethyl)

To a solution of the compound IVR 0.19 g (0.84 mmol) obtained in Example 20 and ethyl acetate 2 ml while stirring at room temperature, diphenyl diazomethane 0.17 g (0.87 mmol) in ethyl acetate (2 ml) solution is added. The mixture is stirred for 1 hour. After completion of the reaction, the solvent is distilled off under reduced pressure. To the residue n-hexane is added to crystallize the desired product. By filtration, the crystals are obtained on the filter paper, and washed with a small portion of n-hexane, and dried under reduced pressure to give the title product IV″R as white crystals, yielding 0.29 g (87%).
Specific rotation $[\alpha]_D$ = – 34.0° (C = 0.20, $CHCl_3$)
TLC, Rf = 0.70 (ethyl acetate : n-hexane = 1 : 2)
$^1$H-NMR (90 MHz ; $CDCl_3$) δ ppm : 1.63 (S, 6 H), 3.31 (d, 1 H), 5.03 (d, 1 H), 6.5-7.2 (m, 14 H)

Example 22 (S)-2-(3,4-0-Isopropylidenedioxyphenyl)-2-phthalimidoxy acetic acid diphenyl-methyl ester (VII : B = isopropylidene, $R_1$ = diphenylmethyl)

To the compound IV″R 50 mg (.12 mmol) as obtained in Example 21, N-hydroxyphthalimide 1.9 mg (0.11 mmol) and triphenylphosphine 34 mg (0.12 mmol) in THF (0.5 ml) solution while cooling and stirring in an ice-water bath, azodicarboxylic acid diethyl ester 24 μl (0.15 mmol) in THF (0.5 ml) solution is added. The mixture is stirred for 3 hours. Ethyl acetate 50 ml is added, and the solution is washed with diluted $NaHCO_3$ aqueous solution (20 ml × twice) and saturated NaCl (20 ml × twice), and dried over anhydrous magnesium sulfate. The

solvent is distilled off under reduced pressure. The residue is purified by silica gel chromatography to give in the ethyl acetate n-hexane (1 : 2) fraction the title compound as a white solid, yielding 30 mg (43%).
Specific rotation $[\alpha]_D$ = – 120.0° (C = 0.12, $CHCl_3$)
TLC, Rf = 0.53 (silica gel/ethyl acetate : n-hexane = 1 : 2)
$^1$H-NMR (90 MHz ; $CDCl_3$) δ ppm : 1.61 (6 H, S), 5.80 (1 H, S), 6.46-7.13 (14 H, m), 7.56 (4 H, m)

Example 23 2-(2-aminothiazole-4-yl)-2-[(S)-α-tertbutyloxycarbonyl-3,4-0-isopropylidenedioxy benzyloxyimino] acetic acid (syn form) (X : B = isopropylidene, $R_1$ = tert-butyl, $R_2$ = hydrogen)

(S)-2-(3,4-0-Isopropylidenedioxyphenyl)-2-aminooxy acetic acid tert-butyl ester (VIII) 0.45 g (1.52 mmol) is dissolved in ethanol 20 ml, and 2-amino-thiazole-4-ylglyoxylic acid (IX) 0.33 g (1.9 mmol) is added. The mixture is stirred for 5 hours at room temperature. After completion of the reaction, the solvent is distilled off under reduced pressure to conetrate the reaction solution to about one quarter volume. The mixture is poured into ice-water 50 g and the thus precipitated crystals are separated by filtation, and washed with water 50 ml, and dried under reduced pressure. The filtrate and the washed solution are combined and therefrom the desired product is extracted with ethyl acetate (30 ml). The organic layer is washed with water (20 ml × twice) and dried over anhydrous magnesium sulfate. The solvent is distilled off under reduced pressure. The thus obtained residue and the previously obtained crystals are combined, and dissolved in acetone 30 ml. Insoluble impurities are removed by filtration. From the filtrate the solvent is distilled off under reduced pressure to give the title compound X as yellow solid, yielding 0.48 g (70%).
Specific rotation $[\alpha]_D$ = + 45.0° (C = 0.20, acetone)
M.P. 111°C (decomposition)
TLC, Rf = 0.33 (silica gel/methanol : chloroform : 1. 3)
$^1$H-NMR (90 MHz, DMSO-$d_6$) δ ppm : 1.40 (9 H, S), 1.63 (6 H, S), 5.35 (1 H, S), 6.8 (4 H, m) 7.13 (2 H, b)
IR spectrum (KBr, tablet) ($cm^{-1}$) 3400, 2950, 2970, 1735, 1625, 1500, 1445, 1375, 1260, 1220, 1155

Example 24 2-(2-aminothiazol-4-yl)-2-(α-tert-butyloxycarbonyl-3,4-0-cyclohexylidene dioxybenzyloxyimino) acetic acid (syn form) (X : B = cyclohexylidene, $R_1$ = tert-butyl, $R_2$ = hydrogen)

2-(3.4-0-cyclohexylidenedioxyphenyl)-2-aminooxy acid acid tert-butyl ester (VIII) 70 mg (0.18 mmol) is dissolved in methanol 5 ml, and 2-aminothiazole-4-ylglyoxylic acid (IX) 30 mg (0.17 mmol) is added. The mixture is stirred for 2 hours at room temperature. After completion of the reaction, ice-water 10 g is added to the mixture, and the desired product is extracted with ethyl acetate 40 ml. The organic layer is washed with saturated NaCl 20 ml, and dried over anhydrous magnesium sulfate. The solvent is distilled off under reduced pressure to obtain the title compound as a yellow solid, yielding 79 mg (95%).
TLC, Rf = 0.35 (silica gel/methanol : chloroform = 1 : 3)
$^1$H-NMR (90 MHz, DMSO-$d_6$) δ ppm : 1.40 (9 H, S), 1.3-2.0 (10 H, m) 5.35 (1 H, S), 6.7 (4 H, m) 7.1 (2 H, b)
IR spectrum (KBr, tablet) ($cm^{-1}$) 2940, 2860, 1735, 1625, 1500, 1450, 1370, 1250, 1150

Example 25 (S)-2-(3,4-0-isopropylidenedioxyphenyl)-2-aminooxy acetic acid diphenyl-methyl ester (VIII : B = isopropylidene, $R_1$ = diphenylmethyl)

The compound VII 2.65 g (4.95 mmol) as obtained in Example 22 is dissolved in methylene chloride 40 ml. To the mixture while cooling and stirring in an ice-water bath, hydrazine hydrate 0.48 ml (9.90 mmol) is added, and the mixture is stirred for 30 minutes. After completion of the reaction, the precipitated material is separated by filtration. The filtrate is washed twice with water 10 ml, and dried over anhydrous magnesium sulfate. The solvent is distilled off under reduced pressure. To the residue n-hexane 50 ml is added, and the mixture stirred under cooling in an ice-water bath for 1 hour. The precipitated material is obtained on the filter paper and dried under reduced pressure to give the title compound VIII as white crystals, yielding 1.65 g (75%).
$^1$H-NMR (90 MHz ; $CDCl_3$) δ ppm : 1.60 (6 H, S), 5.08 (1 H, S), 5.68 (2 H, b), 6.53-6.81 (3 H, m), 6.86 (1 H, S), 6.96-7.30 (10 H, m)

Example 26 2-(2-aminothiazol-4-yl)-2-[(S)-α-diphenylmethyloxycarbonyl-3,4-0-isopropylidendioxy benzyloxyimino] acetic acid (syn form) (X : B = isopropylidene, $R_1$ = diphenylmethyl, $R_2$ = hydrogen)

The compound VIII 0.52 g (1.28 mmol) as obtained in Example 25, is dissolved in methanol 15 ml, and 2-aminothiazole-4-yl glyoxylic acid (IX) 0.22 g (1.27 mmol) is added. The mixture is stirred for 1 hour at room temperature. The solvent is distilled off under reduced pressure and the thus obtained residue is dissolved in

acetone 13 ml. To the mixture under cooling in an ice-water bath, β-phenylethylamine 0.15 ml (1.19 mmol) is added. The mixture is stirred for 1 hour. The precipitated matieral is obtained on the filter paper, and washed with acetone 5 ml, and then dispersed in a mixture of ethyl acetate (30 ml) and water (30 ml). The mixture is adjusted to pH 1.0 by the addition of 1 N HCl thereto while cooling and stirring in the ice-water bath. The organic layer is separated, and then washed with saturated NaCl, and dried over anhydrous magnesium sulfate. The solvent is distilled off under reduced pressure to obtain the title compound (X) as pale yellow crystals, yielding 0.54 g (73%).
TLC, Rf = 0.62 (silica gel/methanol : chloroform = 1 : 3)
Specific rotation $[\alpha]_D$ = +18.8° (C = 0.55, acetone)
$^1$H-NMR (90 MHz, DMSO-$d_6$) δ ppm : 1.63 (6 H, S), 5.71 (1 H, S), 6.8 (5 H, m), 7.2 (12 H, m)

Example 27 2-(2-triphenylmethylaminothiazol-4-yl)-2-(α-tert-butyloxycarbonyl-3,4-0-isopropylidene dioxy benzyloxyimino) acetic acid (syn form) (X : B = Isopropylidene, $R_1$ = tert-butyl, $R_2$ = triphenylmethyl)

To an ethanol (7 ml) solution of 2-(3,4-0-isopropylidenedioxyphenyl)-2-aminooxy acetic acid tert-butyl ester (VIII) 0.11 g (0.37 mmol) under stirring at room temperature, 2-triphenylmethylaminothiazole-4-ylglyoxylic acid (IX) 0.14 g (0.34 mmol) is added. The mixture is stirred for 3 hours at room temperature. After completion of the reaction, ethyl acetate (50 ml) is added. The mixture is washed with ice-water (30 g), dilute citric acid solution (30 ml) and saturated NaCl (30 ml × twice), and dried over anhydrous magnesium sulfate. The solvent is distilled off under reduced pressure to give the title product as a yellow solid yielding 0.19 g (70%).
TLC, Rf = 0.55 (silica gel/methanol : ethyl acetate = 1 : 5)
$^1$H-NMR (90 MHz, DMSO-$d_6$) δ ppm : 1.36 (9 H, S), 1.63 (6 H, S), 5.30 (1 H, S) 6.76 (4 H, m) 7.26 (15 H, m), 8.66 (1 H, S)
IR spectrum (KBr, tablet) ($cm^{-1}$) 3420, 2980, 2930, 1735, 1625, 1595, 1530, 1500, 1450, 1375, 1255, 1155, 700

Example 28 2-(2-triphenylmethylaminothiazole-4-yl)-2-(α-diphenylmethyloxycarbonyl-3,4-0-isopropylidenedioxybenzyloxyimino) acetic acid (synform) (X : B = isopropylidene, $R_1$ = $Ph_2CH$—, $R_2$ = $Ph_3C$—)

To a dichloromethane (3 ml) solution of 2-(3,4-0-isopropylidenedioxyphenyl)-2-phthalimidoxy acetic acid diphenylmethyl ester (VII) 90 mg (0.17 mmol), while cooling and stirring in an ice-water bath, hydrazine hydrate 17 μl (0.35 mmol) is added, and the mixture is stirred for 30 minutes. After completion of the reaction, anhydrous magnesium sulfate (3 g) is added. The mixture is stirred for 5 minutes at room temperature, and then filtered. From the filtrate the solvent is distilled off under reduced pressure, and to the thus obtained residue ethanol (3 ml) is added. To the mixture while stirring at room temperature, 2-triphenylmethylaminothiazole-4-ylglyoxylic acid (IX) 62 mg (0.15 mmol) is added. After 3 hours stirring, ethyl acetate (50 ml) is added to the above mixture. The mixture is washed with ice-water (30 g), dilute citric acid solution (20 ml) and saturated NaCl (20 ml × twice), and then dried over anhydrous magnesium sulfate. The solvent is distilled off to obtain the title compound as a yellow solid yielding 72 mg (60%).
TLC, Rf = 0.55 (silica gel/methanol : ethyl acetate = 1 : 5)
$^1$H-NMR (90 MHz, DMSO-$d_6$) δ ppm : 1.61 (6 H, S), 5.66 (1 H, S), 6.78 (5 H, m), 7.2 (25 H, m), 8.71 (1 H, S)
IR spectrum (KBr, tablet) ($cm^{-1}$) 3430, 3060, 3030, 2990, 2930, 2860, 1740, 1625, 1600, 1530, 1500, 1450, 1380, 1255, 1190, 700

Example 29
2-(2-formylaminothiazol-4-yl)-2-(α-tert-butyloxycarbonyl-3,4-0-isopropylidenedioxybenzyloxyimino) acetic acid (syn form) (X ; B = isopropylidene, $R_1$ = tert-butyl-, $R_2$ = CHO)

To an ethanol (10 ml) solution of the compound VIII 0.50 g (1.69 mmol) obtained in Example 11, 2-formyl amino thiazol-4-yl glyoxylic acid 0.34 g (1.68 mmol) is added. The mixture is stirred while heating to 40°C for 4 hours in an oil bath. After completion of the reaction, insoluble impurities are removed by filtration. To the filtrate ethyl acetate (50 ml) is added, and the mixture is washed with ice-water (30 g), dilute HCl aqueous solution (30 ml × twice) and saturated NaCl (20 ml × twice), and then dried over anhydrous magnesium sulfate. The solvent is distilled off under reduced pressure to obtain the title compound (X) as a pale yellow solid, yielding 0.40 g (50%).
TLC ; Rf = 0.68 (silica gel/methanol : chloroform = 1 : 3)
$^1$H-NMR (90 MHz, DMSO-$d_6$) δ ppm 1.41 (9 H, S), 1.65 (6 H, S), 5.43 (1 H, S), 6.83 (3 H, m), 7.48 (1 H, S),

8.46 (1 H, S), 12.56 (1 H, b)
IR spectrum (KBr, tablet) ($cm^{-1}$) 3430, 2908, 2930, 1740, 1700, 1550, 1500, 1445, 1375, 1280, 1260, 1220, 1155.

Referential Example 1 M-14659 :
7-[2-(2-aminothiazol-4-yl)-2-(S)-(α-carboxy-3,4-dihydroxybenzyloxyimino) acetamide]-3-(2-carboxy-5-methyl-S-triazolo [1,5-a] pyrimidine-7-ylthiomethyl)-3-cephem-4-carboxylic acid sodium salt (syn form) (XIII)

The compound X 260 mg (0.58 mmol) as obtained in Example 23 is dissolved in THF 2 ml and to this solution methylene chloride (6 ml) solution of 7-amino-3-(2-diphenylmethyloxycarbonyl-5-methyl-S-triazolo [1,5-a] pyrimidine-7-ylthiomethyl)-3-cephem-4-carboxylic acid diphenylmethyl ester (XI) 436 mg (0.58 mmol) is added. To this mixture under cooling with ice, a THF solution 4 ml of DCC 143 mg (0.70 mmol) is added. It is stirred for 1 hour under cooling with ice, and then for 21 hours at room temperature, and then filtered. Ethylacetate is added to the filtrate. It is washed with $NaHCO_3$ aqueous solution, and then saturted NaCl, and dried over anhydrous magnesium sulfate. The solvent is distilled off under reduced pressure. The residue is purified by silica gel column chromatography ($SiO_2$ 35 g, n-hexane : ethyl acetate = 1 : 5) to obtain the desired condensation product XII (B = isopropylidene, $R_1$ = tert-butyl, $R_2$ = hydrogen), yielding 270 mg (39%).
$^1$H-NMR (400 MHz, $CDCl_3$) δ ppm : 1.33 (9 H, S), 1.64 (6 H, d), 2.51 (3 H, S), 3.60 (2 H, S), 4.28 (1 H, d), 4.43 (1 H, d), 5.10 (1 H, d), 5.67 (1 H, S), 5.94 (1 H, m), 6.69 (1 H, d), 6.83 (1 H, S), 6.94 (3 H, m), 7.02 (1 H, S), 7.22-7.51 (21 H, m) 9.08 (1 H, d)
TLC, Rf = 0.40 (silica gel/ethyl acetate)

The thus obtained condensation product (XII) 250 mg is dissolved in anisole 2 ml, and to this mixture while cooling with ice, trifluoroacetic acid 4 ml is added. The mixture is stirred at room temperature. After stirring for four hours, ether/n-hexane (2 : 1) solution 18 ml is added thereto under cooling with ice. The thus precipitated crystals are obtained by filtration, and dried, and then suspended in water 5 ml. The suspension is adjusted to pH 6.5 by adding $NaHCO_3$ aqueous solution. The mixture is purified through HP-20 resin (Trade Name) to obtain the antibiotic substance, M-14659 (XIII), yielding 68 mg (39%)
$^1$H-NMR (400 MHz, DMSO-$d_6$ + $D_2O$) δ ppm 2.59 (3 H, S), 3.38 (1 H, d), 3.58 (1 H, d) 4.24 (1 H, d), 4.75, (1 H, d), 5.00 (1 H, d), 5.11 (1 H, S), 5.64 (1 H, d), 6.67 (1 H. d), 6.78 (1 H, S), 6.88 (2 H, m), 7.48 (1 H, S)

## Claims

1. A phenylacetic acid derivative represented by the following general formula :

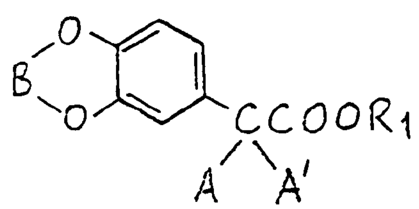

wherein A and A′ together stand for an oxygen atom, or A′ stands for hydrogen while A stands for hydroxy, halo, phthalimidoxy, aminooxy, or a substituent of the formula :

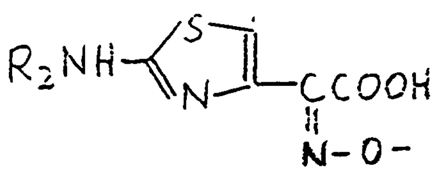

$R_1$ stands for hydrogen or a carboxyl protecting group ; B stands for a phenolic hydroxy protecting group ; $R_2$ stands for a hydrogen atom or an amino protecting group ; and wherein the phenylacetic acid derivative is other than 2-[α-benzhydryloxy carbonyl-3,4-di(2-methoxyethoxymethoxy)-benzyloxyimino]-2-(tritylaminothiazol-4-yl) acetic acid and other than 2-(2- triphenylmethylaminothiazol-4-yl)-2-(α-carboxyl-3,4-0-isopropylidenedioxybenzyloxyimino) acetic acid.

2. A compound of claim 1 wherein B is selected from ethylidene, propylidene, butylidene, pentylidene, cyclopentylidene, cyclohexylidene, two alkyl groups and two aralkyl groups.

3. A compound of claim 1 or claim 2, wherein said carboxyl protecting group is selected from t-butyl, diphenylmethyl, p-methoxybenzyl and p-nitrobenzyl.

4. A compound of any one of claims 1, 2 and 3 wherein said amino protecting group is a trityl group or a formyl group.

5. A process which comprises the use of a compound of any one of claims 1 to 4 in the preparation of cephalosporin antibiotic substances.

6. A process according to claim 5 which involves the preparation of antibiotic M-14659 of the formula :

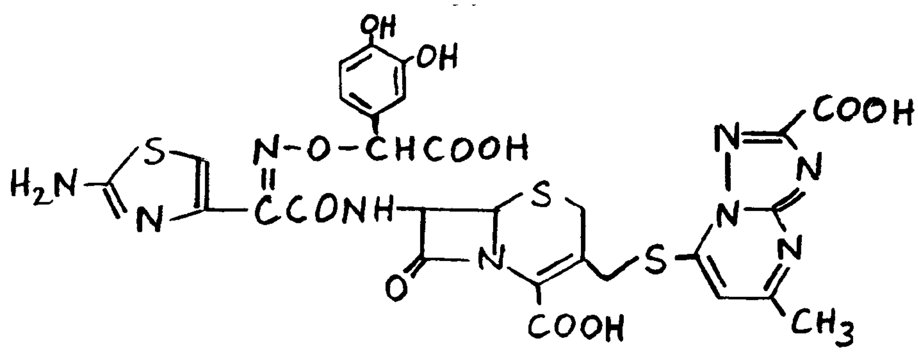

7. A process according to claim 5 or claim 6 wherein a compound of claim 1 in which A′ is hydrogen and A is

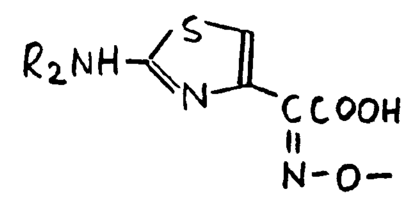

is reacted with a compound of the formula

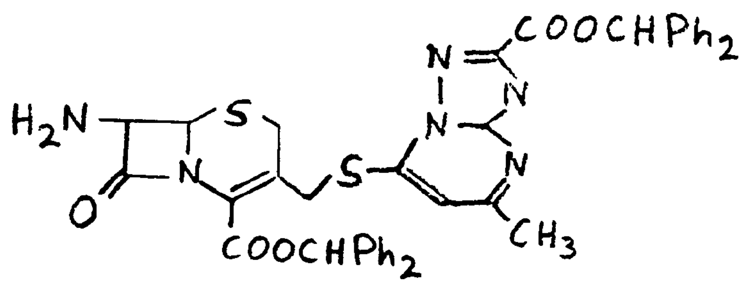

by condensation of their —COOH and $H_2N$— groups respectively.

8. A process according to claim 7 in which the condensation is followed by removal of the protecting groups $R_1$, $R_2$ and B to give antibiotic M-14659.

9. A process according to any one of claims 5 to 8 wherein an optically active compound of claim 1 is used.

10. A process according to claim 9 wherein the optically active compound of claim 1 has the (S) configuration at the chiral centre.

11. A process which comprises the preparation of a compound of any one of claims 1 to 4.

12. A process according to claim 11 which comprises one or more steps selected from :

(i) hydrolysing a compound of the formula :

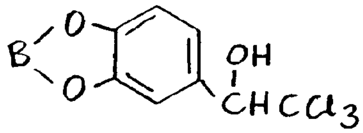

(ii) reacting a protected catechol of the formula

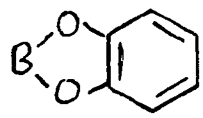

with an alkyl chloroformyl formate to produce a compound of claim 1 in which A and A′ together are an oxygen atom ;
(iii) reducing a compound of claim 1 in which A and A′ together are an oxygen atom to produce a compound of claim 1 in which A′ is hydrogen and A is hydroxy ;
(iv) halogenating a compound of claim 1 in which A′ is hydrogen and A is hydroxy to produce the corresponding compound in which A is a halogen atom ;
(v) reacting a compound of claim 1 in which A′ is hydrogen and A is halo or hydroxy with N-hydroxyphthalimide to produce the corresponding compound in which A is a phthalimidoxy group ;
(vi) forming an ester of a compound of claim 1 in which $R_1$ is hydrogen ;
(vii) producing a compound of claim 1 in which $R_1$ is hydrogen by hydrolysis of a corresponding carboxylic acid ester ;
(viii) converting the phthalimidoxy group of a phthalimidoxy compound of claim 1 to an aminooxy group ;
(ix) reacting an aminooxy compound of claim 1 with 2-aminothiazol-4-yl glyoxylic acid to prepare a compound of claim 1 in which A′ is hydrogen and A is

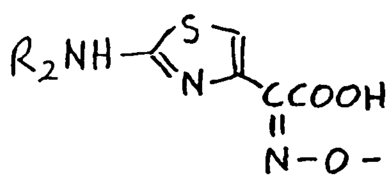

(x) resolving the optical isomers of a compound of claim 1 ;
(xi) resolving the optical isomers of a compound of claim 1 and isolating the (S) stereoisomer ;
(xii) reducing a compound of claim 1 in which A and A′ together are an oxygen atom under conditions such as to produce a single stereoisomer ;
(xiii) reducing a compound of claim 1 in which A and A′ together are an oxygen atom with yeast or with sodium borohydride in the presence of L-proline to produce the (R) stereoisomer ;
(xiv) preparing an (S) stereoisomer of a phthalimidoxy compound of claim 1 by introduction of the phthalimidoxy group into a compound of claim 1 having the (R) configuration.

## Patentansprüche

1. Phenylessigsäurederivat der folgenden allgemeinen Formel

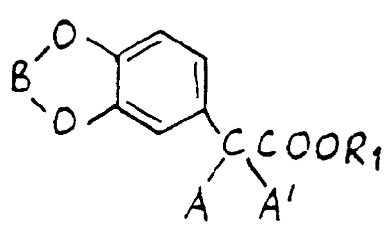

wobei A und A′ gemeinsam ein Sauerstoffatom bedeuten oder A′ Wasserstoff bedeutet, während A Hydroxy,

Halogen, Phthalimidoxy, Aminooxy oder einen Substituenten der Formel

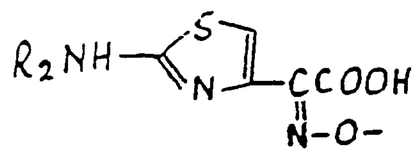

bedeutet, wobei $R_1$ Wasserstoff oder eine Carboxylschutzgruppe bedeutet ; B eine phenolische Hydroxylschutzgruppe bedeutet ; $R_2$ ein Wasserstoffatom oder eine Aminoschutzgruppe bedeutet ; und wobei das Phenylessigsäurederivat nicht 2-[α-Benzhydryloxycarbonyl-3,4-di (2-methoxyethoxymethoxy)-benzyloxyimino]-2-(tritylaminothiazol-4-yl) essigsäure und nicht 2-(2-Triphenylmethylaminothiazol-4-yl)-2-(α-carboxyl-3,4-0-isopropylidendioxybenzyloxyimino)essigsäure ist.

2. Verbindung nach Anspruch 1, wobei B aus Ethyliden, Propyliden, Butyliden, Pentyliden, Cyclopentyliden, Cyclohexyliden, zwei Alkylgruppen und zwei Aralkylgruppen ausgewählt ist.

3. Verbindung nach Anspruch 1 oder Anspruch 2, wobei die Carboxylschutzgruppe aus t-Butyl, Diphenylmethyl, p-Methoxybenzyl und p-Nitrobenzyl ausgewählt ist.

4. Verbindung nach einem der Ansprüche 1, 2 und 9, wobei die Aminoschutzgruppe eine Tritylgruppe oder eine Formylgruppe ist.

5. Verfahren, welches die Verwendung einer Verbindung nach einem der Ansprüche 1 bis 4 bei der Herstellung von antibiotischen Cephalosporinsubstanzen umfaßt.

6. Verfahren nach Anspruch 5, welches die Herstellung des Antibiotikums M-14659 der Formel

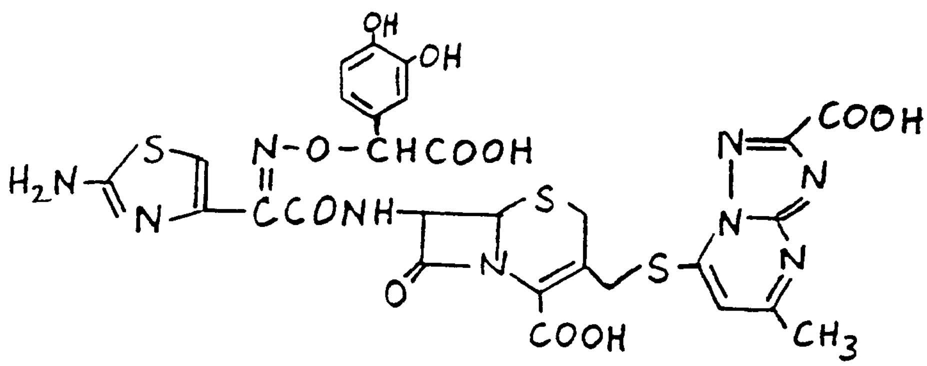

umfaßt.

7. Verfahren nach Anspruch 5 oder Anspruch 6, wobei eine Verbindung nach Anspruch 1, in der A′ Wasserstoff und A

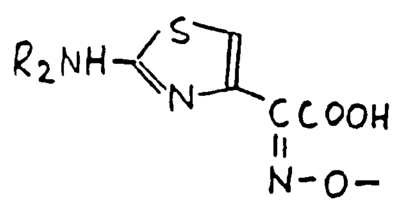

ist, mit einer Verbindung der Formel

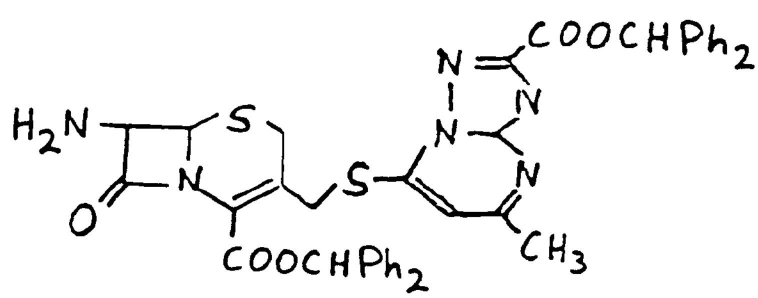

durch Kondensation ihrer jeweiligen —COOH— und $H_2N$-Gruppen umgesetzt wird.

8. Verfahren nach Anspruch 7, wobei nach der Kondensation die Schutzgruppen $R_1$, $R_2$ und B entfernt werden, um das Antibiotikum M-14659 zu ergeben.

9. Verfahren nach einem der Ansprüche 5 bis 8, wobei eine optisch aktive Verbindung nach Anspruch 1 eingesetzt wird.

10. Verfahren nach Anspruch 9, wobei die optisch aktive Verbindung nach Anspruch 1 im Chiralitätszentrum die (S)-Konfiguration hat.

11. Verfahren, das die Herstellung einer Verbindung nach einem der Ansprüche 1 bis 4 umfaßt.

12. Verfahren nach Anspruch 11, das eine oder mehrere der aus den folgenden Stufen ausgewählten Stufen umfaßt :

(i) Hydrolysieren einer Verbindung der Formel :

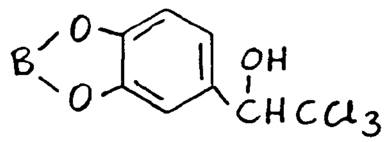

(ii) Umsetzen eines geschützten Catechols der Formel

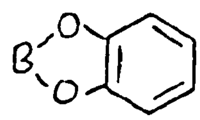

mit einem Alkylchlorformylformiat zur Gewinnung einer Verbindung nach Anspruch 1, in der A und A′ gemeinsam ein Sauerstoffatom sind ;

(iii) Reduzieren einer Verbindung nach Anspruch 1, in der A und A′ gemeinsam ein Sauerstoffatom sind, unter Bildung einer Verbindung nach Anspruch 1, in der A′ Wasserstoff und A Hydroxy ist ;

(iv) Halogenieren einer Verbindung nach Anspruch 1, in der A′ Wasserstoff und A Hydroxy ist, zur Bildung der entsprechenden Verbindung, in der A ein Halogenatom ist ;

(v) Umsetzen einer Verbindung nach Anspruch 1, in der A′ Wasserstoff und A Halogen oder Hydroxy ist, mit N-Hydroxyphthalimid zur Bildung der entsprechenden Verbindung, in der A eine Phthalimidoxygruppe ist ;

(vi) Bilden eines Esters einer Verbindung nach Anspruch 1, in der $R_1$ Wasserstoff ist ;

(vii) Herstellen einer Verbindung nach Anspruch 1, in der $R_1$ Wasserstoff ist, durch Hydrolyse des entsprechenden Carbonsäureesters ;

(viii) Umwandeln der Phthalimidoxygruppe einer Phthalimidoxyverbindung nach Anspruch 1 in eine Aminooxygruppe ;

(ix) Umsetzen einer Aminooxyverbindung nach Anspruch 1 mit 2-Aminothiazol-4-yl-glyoxylsäure zur Bildung einer Verbindung nach Anspruch 1, in der A′ Wasserstoff und A

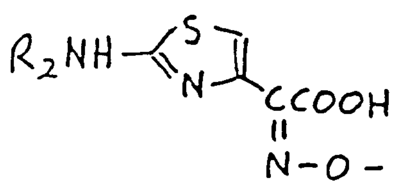

ist ;

(x) Trennung der optischen Isomeren einer Verbindung nach Anspruch 1 ;

(xi) Trennung der optischen Isomeren einer Verbindung nach Anspruch 1 und Isolieren des (S)-Stereoisomeren ;

(xii) Reduzieren einer Verbindung nach Anspruch 1, in der A und A′ gemeinsam ein Sauerstoffatom sind, unter solchen Bedingungen, daß ein einziges Stereoisomer erzeugt wird ;

(xiii) Reduzieren einer Verbindung nach Anspruch 1, in der A und A′ gemeinsam ein Sauerstoffatom sind, mit Hefe oder Natriumborhydrid in Gegenwart von L-Prolin zur Bildung des (R)-Stereoisomeren ;

(xiv) Herstellen eines (S)-Stereoisomeren aus einer Phthalimidoxyverbindung nach Anspruch 1 durch Einführen der Phthalimidoxygruppe in eine die (R)-Konfiguration aufweisende Verbindung nach Anspruch 1.

## Revendications

1. Un dérivé d'acide phénylacétique représenté par la formule générale suivante :

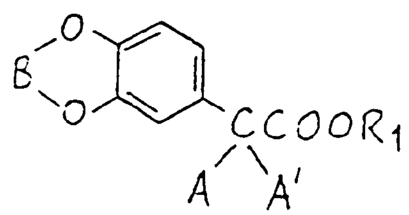

dans laquelle A et A′ ensemble représentent un atome d'oxygène, ou bien A′ désigne l'hydrogène tandis que A désigne un groupe hydroxy, halo, phtalimidoxy, aminooxy ou un substituant de formule :

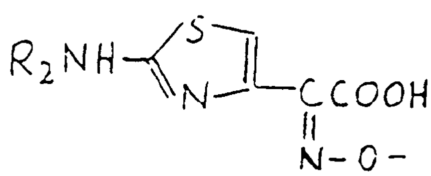

$R_1$ désigne un hydrogène ou un groupe protecteur du groupe carboxyle ; B désigne un groupe protecteur du groupe hydroxy phénolique ; $R_2$ désigne un atome d'hydrogène ou un groupe protecteur du groupe amino ; et le dérivé d'acide phénylacétique n'est pas l'acide 2-[α-benzhydryloxycarbonyl-3,4-di(2-méthoxyéthoxyméthoxy)-benzyloxyimino]-2-(tritylaminothiazole-4-yl) acétique et n'est pas l'acide 2-(2-triphénylméthylaminothiazole-4-yl)-2-(α-carboxyl-3,4-0-isopropylidènedioxybenzyloxylimino) acétique.

2. Un composé selon la revendication 1, selon lequel B est choisi parmi les suivants : éthylidène, propylidène, butylidène, pentylidène, cyclopentylidène, cyclohexylidène, deux groupes alkyle et deux groupes aralkyle.

3. Un composé selon la revendication 1 ou 2, selon lequel ledit groupe protecteur du groupe carboxyle est choisi parmi les suivants : t-butyle, diphénylméthyle, p-méthoxybenzyle et p-nitrobenzyle.

4. Un composé selon l'une quelconque des revendications 1, 2 et 3 selon lequel le groupe protecteur du groupe amino est un groupe trityle ou un groupe formyle.

5. Un procédé qui comprend l'utilisation d'un composé de l'une quelconque des revendications 1 à 4 dans la préparation des substances antibiotiques céphalosporines.

6. Un procédé selon la revendication 5, qui comporte la préparation de l'antibiotique M-14659 de formule:

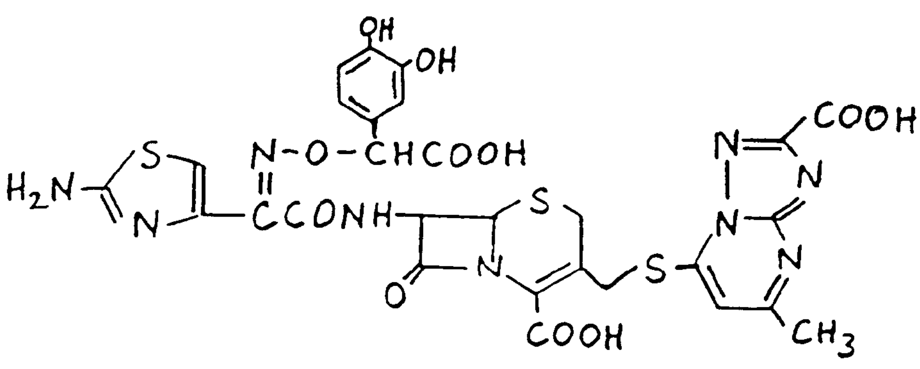

7. Un procédé selon la revendication 5 ou 6, selon lequel un composé selon la revendication 1 dans lequel A′ est un atome d'hydrogène et A est

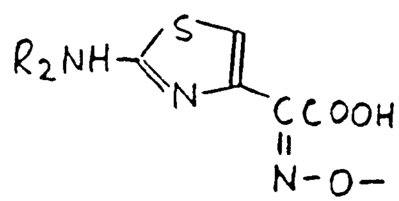

est mis à réagir avec un composé de formule :

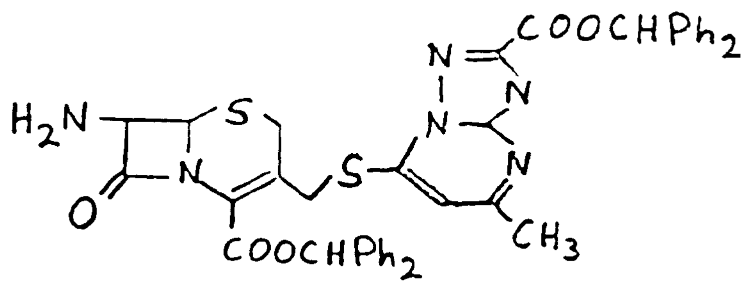

par condensation de leurs groupes —COOH et $H_2N$— respectivement.

8. Un procédé selon la revendication 7, selon lequel la condensation est suivie de l'élimination des groupes protecteurs $R_1$, $R_2$ et B pour donner l'antibiotique M-14659.

9. Un procédé selon l'une quelconque des revendications 5 à 8, selon lequel un composé optiquement actif selon la revendication 1 est utilisé.

10. Un procédé selon la revendication 9, selon lequel le composé optiquement actif selon la revendication 1 a la configuration (S) au centre chiral.

11. Un procédé qui comprend la préparation d'un composé selon l'une quelconque des revendications 1 à 4.

12. Un procédé selon la revendication 11, qui comprend une ou plusieures étapes choisies parmi les suivantes :

(i) hydrolyse d'un composé de formule :

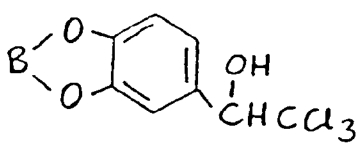

(ii) réaction d'un catéchol protégé de formule :

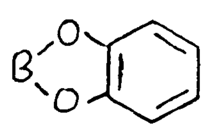

avec un chloroformylformiate d'alkyle pour produire un composé selon la revendication 1, dans lequel A et A′ ensemble représentent un atome d'oxygène ;

(iii) réduction d'un composé selon la revendication 1, dans lequel A et A′ ensemble désignent un atome d'oxygène pour produire un composé selon la revendication 1, dans lequel A′ est l'hydrogène et A est un groupe hydroxy ;

(iv) halogénation d'un composé selon la revendication 1, selon lequel A′ est l'hydrogène et A est un groupe hydroxy pour produire le composé correspondant dans lequel A est un atome d'halogène ;

(v) réaction d'un composé selon la revendication 1, dans lequel A′ est l'hydrogène et A est halo ou hydroxy avec le N-hydroxyphtalimide pour produire le composé correspondant dans lequel A est un groupe phtalimidoxy ;

(vi) formation d'un ester d'un composé selon la revendication 1, dans lequel $R_1$ est l'hydrogène ;
(vii) production d'un composé selon la revendication 1, dans lequel $R_1$ est l'hydrogène par hydrolyse d'un ester d'acide carboxylique correspondant ;
(viii) conversion du groupe phtalimidoxy d'un composé phtalimidoxy selon la revendication 1 en un groupe aminooxy ;
(ix) réaction d'un composé aminooxy selon la revendication 1, avec l'acide 2-aminothiazole-4-yl glyoxylique pour préparer un composé selon la revendication 1 dans lequel A' est l'hydrogène est A est

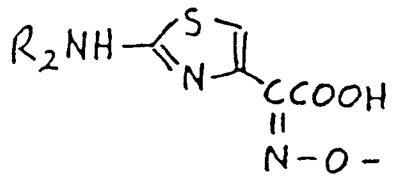

(x) résolution des isomères optiques d'un composé selon la revendication 1 ;
(xi) résolution des isomères optiques d'un composé selon la revendication 1, et isolement du stéréoisomère (S) ;
(xii) réduction d'un composé selon la revendication 1, dans lequel A et A' ensemble désignent un atome d'oxygène dans des conditions telles pour produire un seul stéréoisomère ;
(xiii) réduction d'un composé selon la revendication 1, dans lequel A et A' ensemble désignent un atome d'oxygène, par la levure et le borohydrure de sodium en présence de la L-proline pour produire le stéréoisomère (R) ;
(xiv) préparation d'un stéréoisomère (S) d'un composé phtalimidoxy selon la revendication 1 par l'introduction du groupe phtalimidoxy dans un composé selon la revendication 1 de configuration (R).